# EUROPEAN PATENT SPECIFICATION

(11) **EP 2 219 523 B1**
(45) Date of publication and mention of the grant of the patent: **06.03.2013**
(21) Application number: 08847189.1
(22) Date of filing: 06.11.2008
(51) Int. Cl.: A61B 5/1468

(54) **MEASURING FLUID EXCRETED FROM AN ORGAN**
MESSUNG VON AUS EINEM ORGAN AUSGESCHIEDENER FLÜSSIGKEIT
MESURE DE FLUIDE EXCRÉTÉ PAR UN ORGANE

(30) Priority: 08.11.2007 US 986387 P
(43) Date of publication of application: 25.08.2010
(73) Proprietor: Inolact Ltd., 53251 Givatayim (IL)
(72) Inventor: BINDER, Adina, 75209 Rishon Lezion (IL)
(74) Representative: Sarpi, Maurizio
(86) International application number: PCT/IL2008/001463
(87) International publication number: WO 2009/060448

(56) References cited:
- WO-A1-01/54488
- WO-A1-2005/107580
- WO-A2-2004/049936
- WO-A2-2005/016220
- US-A- 5 827 191
- US-A1- 2005 059 928
- US-A1- 2006 085 049
- US-A1- 2006 241 514
- US-A1- 2006 253 011
- US-B2- 7 184 820

## Description

### CROSS REFERENCE TO RELATED APPLICATION

This application claims the benefit of U.S. Provisional Patent Application No. 60/986,387 filed on November 8, 2007**.**

### FIELD OF THE INVENTION

The present invention generally relates to the field of bio-impedance analysis. More particularly, the present invention relates to measuring characteristics (volume and composition) of fluid excreted from an organ.

### BACKGROUND OF THE INVENTION

In some major medical applications and other various applications, it is very important to measure the quantity, as well as the composition, of fluids that are excreted from the human body. One example is the case of urine, where information on the urination rate and/or urinary bladder state of an individual is essential in assessing his physiological condition. Such information is especially applicable in cases of Diabetes Mellitus, Diabetes Insipidus, SIADH, Acute Renal failure, and others.

In another case, the fluid of interest is breast milk. Many medical authorities worldwide highly recommend to breastfeed the newborn infant for at least a period of the first few months of his life. Medical professionals encourage the mothers to breastfeed their babies, if not fully, at least partially for periods of at least 3 to 6 months from the date of birth. The composition of breast milk changes during the time (days, weeks, months) after giving birth, e.g. the percentage of fat and other ingredients changes in relation to the number of weeks since giving birth and of lactation.

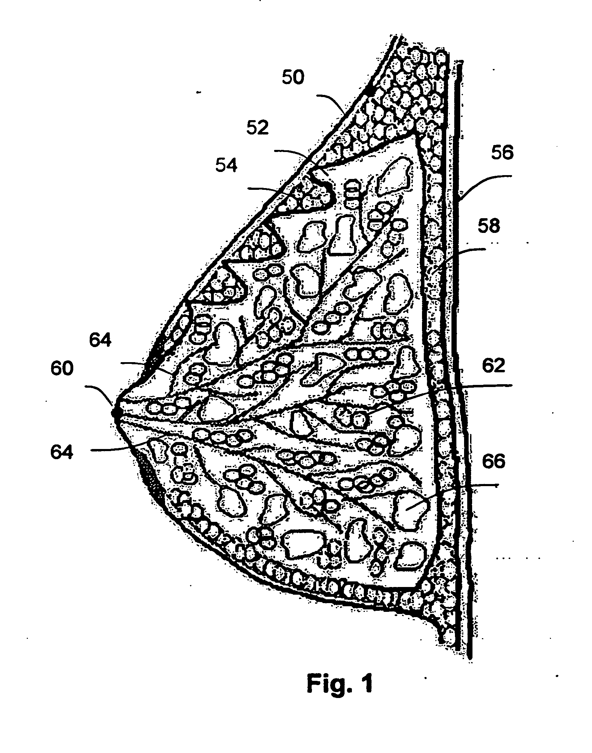

The breast secretory gland is composed of the following parts (Fig. 1): Glandular tissue 66, which produce and transports milk, connective tissue (Cooper's ligaments **52**), which supports the breast, adipose (fatty) tissue 54 (including intraglandular **62**, subcutaneous and retromammary fat **58**), which offers protection from injury, blood, which nourishes breast tissue and provides the nutrients needed to make milk, lymph, which removes waste and nerves, which make the breast sensitive to touch and allow the baby's suck to stimulate the release of hormones that trigger the let-down, or milk ejection, reflex and the production of milk. For bioelectrical studies, the anatomy of the breast can be represented as following: Immediately under the skin **50,** the adipose tissue **54** consists of vesicular cells filled with fat and collected into lobules separated by Cooper ligament **52.** The innermost tissue of the breast consists of mammary gland and connective tissue. Each breast has about 15 to 20 sections termed lobes with many smaller sections termed lobules (glandular lobules and fat lobules). Each section is connected to the nipple 60 by thin tubes called lactiferous ducts **64.** Most breasts contain abundant fatty tissue with a large quantity of non-living intercellular matrix. Internally, the breast is separated from the great pectoral muscle **56** by the retromammary fat **58.**

The 2006 paper "Analysis of human breast milk at microwave frequencies" by Lonappan, A., Thomas, V., Bindu, G. Hamsakutty, V. and Mathew, K.T. - Progress in Electromagnetics research, PIER 60, 179-185, discloses a comprehensive study of the dielectric properties of human breast milk at microwave frequencies. The 2007 paper "How Breastfeeding Works" by Kent, J. C., - J Midwifery Womens Health 52: 564-570, discloses an overview of the lactation process in relation to milk volume and composition and to the baby's health.

It is widely recognized that breastfeeding of babies bares a problem of monitoring of the amount of milk intake by the baby. The most commonly used method for determining the milk volume during breastfeeding involves infant's weight measurement before and after breastfeeding. U.S. Patent No. US5065830, relates to this method and U.S. Patent No. US4972391 discloses constant monitoring of duration and frequency of feeding sessions. Apart from the obvious inconvenience, the volume of milk measured is rather inaccurate, and the data is gathered post feeding.

U.S. Patent No. US5827191 tried to overcome this problem by suggesting a method of monitoring the volume of milk during breast feeding by utilizing a porous elastic nipple shaped cover over the nipple area of a women's breast. A micro measurement sensor is then located in a space between the nipple and the elastic cover to measure the volume of the milk flowing there through. Data from the sensor is gathered and then processed to indicate total milk volume intake by the baby.

Nonetheless, this method has some drawbacks emerging from the presence of a foreign body in the path between the breast feeding mother and the baby. These include a certain amount of inconvenience for the mother and the baby during the breast feeding cycle. Furthermore, the device might require high care, such as frequent cleaning from milk residues and the improbable, but highly hazardous case of which the baby accidentally inhales or swallows small debris from a defective device.

Others have suggested methods for monitoring breast parameters during expression of milk, with the intent of improving the breast pumping process. U.S. Patent Publication No. US 2005/0059928 suggested a breast receiving portion equipped with a module capable of sensing changes in the breast. The sensor which could be optical, acoustical, thermal or electrical is used to identify different processes within the lactation process, which is then used to optimize the operation of a breast pump. WIPO Publication No. WO0154488, discloses an apparatus for determining the amount of human milk supplied to a feeding baby during a breast-feeding session comprising a flow-meter measuring the amount of milk passing through an outlet in a cap. WIPO Publication No. WO2006003655, discloses a device comprising a cap having a nipple-shaped region with a duct, comprising an embedded sensor associated with the duct for measuring milk volume passing through the duct. WIPO Publication No. W02006054287, discloses an apparatus for measuring the quantity of milk consumed by an infant during a breast-feeding session, comprising Ultra-sonic Doppler-Effect transmitters

US application US2006253011, discloses a method of sensing the concentration level of a particular electrolyte in the sweat fluid of a subject. The method provides : a sweat sensor system having sweat fluid absorbing material, a measuring apparatus for sensing the electrical conductivity of sweat fluid absorbed by the absorbing material and producing ionic concentration data for said at least one particular electrolyte, and a user interface connected to the measurement apparatus for interpreting the data to a user.

Therefore a need is recognized for a device which could provide reliable information of the breastfed infant's nutrition, without compromising the mother's or baby's convenience and health.

Bio-impedance is a measure for the resistance of living tissue to an externally applied electric current. Bioelectrical Impedance Analysis (BIA) utilizes bio-impendance measurement in a simple real-time non-invasive technique, which is safe and painless with no side effects. Today, bio-impedance measurements are an important tool used in non-invasive examinations for various uses (diagnostic applications, therapeutic applications, laboratory applications etc.). There are various applications aimed for the whole body or for a specific organ. The most familiar diagnostic application utilizing bio-impedance measurement is "body composition analysis" which outputs the whole body fat mass, body cell mass, and extra-cellular mass. Another application of bio-impedance analysis is called "impedance plethysmography" which refers for volume estimation using impedance measurements. Today impedance plethysmography is used for measuring blood volume, heart stroke volume, detection of vein thrombosis, and more.

### BRIEF SUMMARY

The present invention as defined in independent claims 1 and 11 and their dependent claims, includes a device for and a method of measuring characteristics of fluid excreted from an organ of the human body. One device comprises at least one pair of electrodes; a power source; and a data processing system. One data processing system comprises a power regulation module and a measurement module. The electrodes are connected to the power source via the data processing system and are arranged in an array in contact of the human body. One array is characterized by a predefined spatial arrangement of the electrodes that is related to the form of the fluid excreting organ. At least one electrode is placed in the vicinity of the fluid excreting organ. The power regulation module is arranged to supply the electrodes with an electric current characterized by predefined parameters. The measurement module is arranged to measure characteristics of the excreted fluid from evolved potentials on the human body due to the electric current, in relation to the predefined spatial arrangement of the electrodes in the array.

In embodiments, the device includes additional sensors such as an acoustic sensor; an optical sensor or a temperature sensor. The data processing system is arranged to enhance the measurement of the characteristics of the excreted fluid utilizing data from the additional sensors.

In embodiments, the device includes an apparatus arranged to support and place the electrodes in the array in contact of the human body in vicinity of the fluid excreting organ.

In embodiments, the organ of the human body is a breast and the excreted fluid is breast milk. The device allows a breast feeding mother to control characteristic of fed breast milk.

One method comprises the stages: (i) placing at least one pair of electrodes in an array in contact of the human body; (ii) supplying the electrodes with an electric current characterized by predefined parameters; (iii) measuring evolved potentials on the human body due to the electric current; and (iv) calculating characteristics of the excreted fluid from the measured evolved potentials in relation to the spatial arrangement of the electrodes in the array. At least one electrode is placed in the vicinity of the fluid excreting organ.

### BRIEF DESCRIPTION OF THE DRAWINGS

The subject matter regarded as the invention will become more clearly understood in light of the ensuing description of embodiments herein, given by way of example and for purposes of illustrative discussion of the present invention only, with reference to the accompanying drawings (Figures, or simply "FIGS."), wherein:
Fig. 1 is an illustration of the breast anatomy;
Fig. 2 is a block diagram illustrating three major modules of a system for measuring characteristics of fluid excreted from an organ of the human body, according to some embodiments of the invention;
Fig. 3 is a block diagram illustrating sensors' unit, according to some embodiments of the invention;
Fig. 4 is an illustration of a device for measuring characteristics of fluid excreted from an organ of the human body, according to some embodiments of the invention;
Figures 5A and 5B are illustrations of possible placement locations for electrodes on a lactating breast, according to some embodiments of the invention;
Figures 5C and 5D are illustrations of schematic cross-sections of the lactating breast, showing possible placement of electrodes, according to some embodiments of the invention;
Figures 6A, 6B and 6C are illustrations of an apparatus arranged to support and place electrodes on a breast, according to some embodiments of the invention;
Fig. 7 is a cross section illustration of an appliance for attaching electrodes to a breast, according to some embodiments of the invention;
Fig. 8 is a flowchart illustrating a method of measuring characteristics of fluid excreted from an organ of the human body, according to some embodiments of the invention;
Fig. 9 is a flowchart illustrating a method of measuring characteristics of fluid excreted from an organ of the human body, according to some embodiments of the invention; and
Fig. 10 is a graph summarizing bio-impedance measurements in fat emulsion models, according to some embodiments of the invention.

### DETAILED DESCRIPTIONS OF SOME EMBODIMENTS OF THE INVENTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting. In other instances, well-known methods, procedures, components and circuits have not been described in detail so as not to obscure the teachings of the present disclosure.

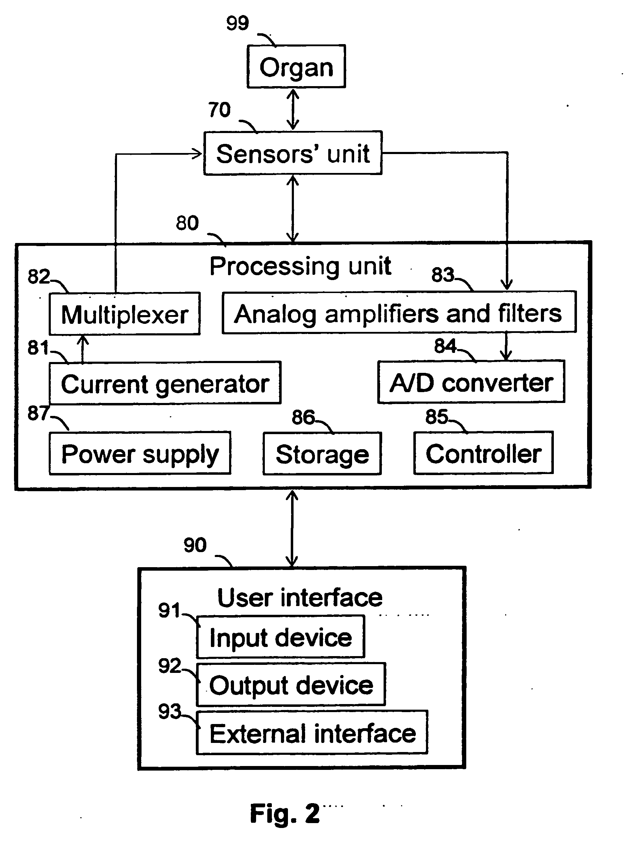

Fig. 2 is a block diagram illustrating three major modules of a system for measuring characteristics of fluid excreted from an organ **99** of the human body, according to some embodiments of the invention. The system comprises a sensors' unit **70,** a processing unit **80** and a user interface **90.** Sensors' unit **70** is connected to processing unit **80,** receives control signals as well as electrical current (e.g. an AC signal) from processing unit **80** and sends measurement (e.g. analog signals) to processing unit **80.** Processing unit **80** is connected to user interface 90 and is operable via user interface **90.**

According to some embodiments of the invention, the modules are presented in a logical manner and may be embodied by multiple, separate or combined physical components. Physical component may embody parts of each module, or parts from different module may be embodied in one component. Connections among the modules may be realized by wire, wireless, by contact or by integration of modules into one module. Modules may be spatially apart and connected via a communication link.

According to some embodiments of the invention, sensors' unit 70 may comprise a patch of electrodes (and possibly other sensors) that may be mounted in contact with organ **99**, such as on the skin of a patient (e.g. on a woman's breast while feeding, skin above the bladder, etc.). While two electrodes may be used to inject low-amplitude alternating current, the recording electrodes may be used to measure the evolving potential changes on the skin surface. The electrodes for applying the electric current and the electrodes for measuring resulting potential differences may be the same electrodes or may be different electrodes for the two tasks. The data from sensors' unit **70** is collected by processing unit **80.** Sensors' unit **70** may comprise other sensors such as an acoustic sensor for identifying baby's milk gulps; a tension sensor, a pulse sensor, a temperature sensor or an optical sensor.

According to some embodiments of the invention, processing unit **80** contains the "brain" and electronics of the system. Processing unit **80** may generate an impedance image of organ **99.** Processing unit **80** is arranged to control and trigger the electric signals' injection and measurement through sensors' unit **70.** Processing unit **80** is arranged to process the collected data and to compute the amount of excreted fluid (e.g. lactated breast-milk, urine, blood, lymph and so forth). Processing unit **80 is** further arranged to output data to user interface **90** (e.g., amount of nursed milk and time duration).

According to some embodiments of the invention, processing unit **80** may comprise a current generator **81** and a multiplexer **82** for providing current to sensors' unit **70** with an electric current. Processing unit **80** may comprise analog amplifiers and filters **83** to amplify organ **99's** reaction to the electric current, e.g. in the form of potential differences, and an analog to digital converter **84** to convert the signal. Processing unit **80** may further comprise a controller **85** such as a CPU (Central Processing Unit), a microcontroller or a DSP (Digital Signal Processor as a specialized microprocessor) for controlling processing unit **80,** and user interface **90.** Processing unit **80** may further comprise a power supply **87** and a storage **86.** Some of the elements of processing unit **80** may be integrated, such as controller **85** with storage **86** and analog to digital converter **84,** or controller **85** with current generator **81.** Power supply **87** may be arranged to feed the whole system.

According to some embodiments of the invention, user interface **90** may comprise a friendly interface for the user (e.g. breastfeeding mother) arranged to present the measured characteristics and statistical data according to infant's age. User interface **90** may comprise an input device **91,** such as command buttons, output device **92** such as a display screen as well as external interface **93,** such as USB or Bluetooth.

According to some embodiments of the invention, either user interface **90,** processing unit **80** or both may be connected to external elements, such as a computer, a computing device, or an electric or mechanic milk pump. The interface with the external elements may be such as to enhance processing and calculations, and/or to improve accuracy by relating to the actual amount of milk excreted.

According to some embodiments of the invention, user interface **90** may be arranged to present data relating to a breast feeding mother and allowing her to control characteristics of milk fed to her baby. The data may further be related to the mother or other mothers (e.g. breast size, number of children), to the baby or other babies (e.g. age, weight), and to the lactation process (e.g. relating to durations, times and frequencies).

According to some embodiments of the invention, the system may be applied to measure urination and urine in the urinary bladder.

According to some embodiments of the invention, two or more electrodes may be used and form, together with organ **99** an electric circuit. Current generator **81** may produce direct current and/or alternating current in one or more frequencies, phases and amplitudes, simultaneously or separately. Either the voltage between the electrodes, or the potential difference to a reference potential (e.g. ground), or both are measured. Different electrode arrays may be used to measure different voltages in a spatial and temporal predefined pattern. Data, such as phase and magnitude, of the generated current as well as the measured voltage may be initially processed to characterize the impedance and other electric properties of the electric circuit. Further data may be collected and utilized to calibrate the device and to generate initialization parameters and initialization data. Data on impedance is then processed together with previously gathered impedance data; with other measured data such as time; and with any type of data measured from other sensors. For example, an acoustic sensor could be utilized in order to identify surges in breast milk flow, which could be identified as baby's milk gulps. Processed information could then be stored from the current breast feeding cycle, for future use; such as long term feeding analysis, and/or presented to any interested party such as the breastfeeding mother, nurse or physician.

According to some embodiments of the invention, evolved potentials, impedance parameters and other electrical properties may be measured in a differential manner. Values may be recorded in relation to a previous state of the breast either prior to the lactation session or at a point set during lactation. This approach promotes reduction in errors.

According to some embodiments of the invention, measurement may be carried out in several frequencies in order to extract the relevant data out of the different measured values. These may include water content of the milk, as opposed to other water containing substances within the breast. In addition, changing masses of fat, protein and carbohydrates within the breast throughout lactation session reflect on their content of the milk, and therefore they may also be measured. According to some embodiments of the invention, recommended frequencies for measurement of evolved potentials are between about 10kHz and 1MHz. However, lower or higher frequencies may also be used.

According to some embodiments of the invention, evolved potentials may be measured by a single array of electrodes, with the objective to record data of a single zone within the breast. In another configuration, evolved potentials could be measured between two or more arrays of electrodes, with the objective of recording evolved potentials and impedance data in discrete zones within the breast. An array of electrodes is defined as a set containing two, three, four or more electrodes.

According to some embodiments of the invention, sampling intervals and pulse length should be small, and long enough, respectively, to acquire continual data on breast's state. On the other hand, the intervals should be maximized under the above requirement in order to minimize interference between signals which might result in accuracy reduction.

According to some embodiments of the invention, calibration of the measuring device is intended to set any parameter required to assess the quantity and/or composition other those who are directly measured by the sensors. Calibration may be appropriate before first use, or periodically, in order to enhance the accuracy of the measurement and/or calculation of the characteristics of the excreted fluid from evolved potentials on the human body due to the electric current. For example, before first use the mother may pump a known quantity of milk, in order for the measuring system to assess parameters that are unique to the mother's anatomical or physiological breast characteristics. Calibration may be carried out using an empirical calibration instrument.

According to some embodiments of the invention, measurements may be stored in storage **86** partly or fully, and be partly or fully used for calculations and later statistical analysis. Either raw data or processed data may be used for the algorithms.

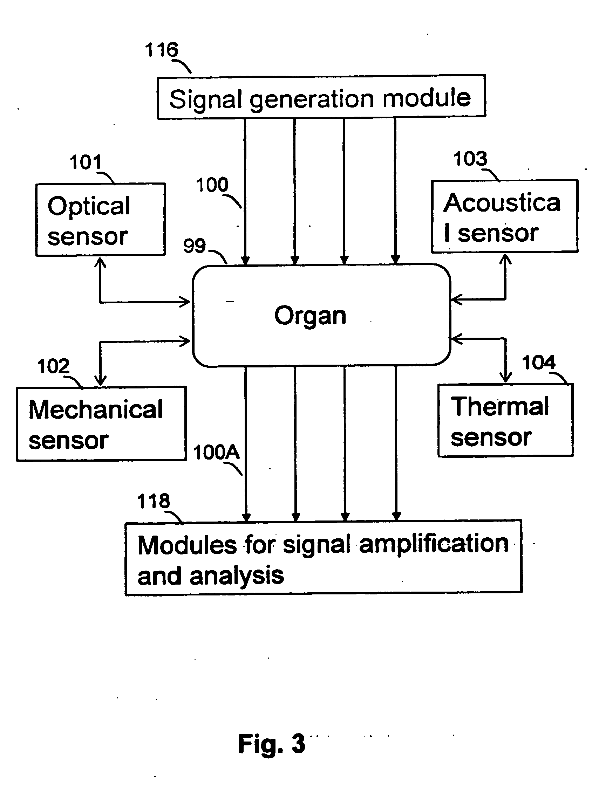

Fig. 3 is at block diagram illustrating sensors' unit **70,** according to some embodiments of the invention. Sensors may comprise electrodes **100,** an optical sensor **101, a** mechanical sensor **102,** an acoustical sensor **103** or a thermal sensor **104.** Any combination of these sensors may be applied, and some sensors may be multiple. Sensors may be integrated with each other (e.g. an thermo-mechanical sensor). Electrodes **100** are generally fed by a signal generation module **116** and signals from electrodes **100A** may be received by modules for signal amplification and analysis **118 -** either or both included e.g. in processing unit **80.** Electrodes **100** used for current injection and electrodes **100A** used for measuring potential differences may be the same electrodes, different electrodes, partially the same electrodes or electrodes **100** and electrodes **100A** may interchange their roles following a temporal pattern. Electrodes **100, 100A** and sensors **101, 102, 103, 104** may be attached to organ 99 in predefined spatial arrangement, and attached or placed in a predefined manner. The separation between electrodes **100** and **100A** in Fig.3 is logical, and must not represent their being physically different and their arrangement. Some or all of electrodes **100** and **100A** may be common electrodes differing temporally in function.

According to some embodiments of the invention, sensors **101, 102, 103, 104** may be utilized to improve the accuracy of measurement. For example, measurements of thermal sensor **104** may be utilize to correct measurements of evolved potentials and impedance parameters according to known dependencies of impedance on temperature for different tissue types and fluids, optical sensor **101,** mechanical sensor **102** or acoustical sensor **103** may be utilized to sense suckle movements, bursts of fluid excretion, position changes or changes in the geometry of the measured organ or of the device. Measurements of evolved potentials and impedance parameters may be corrected accordingly.

According to some embodiments of the invention, sensors **101, 102, 103, 104** may be utilized to initiate or time measurements of evolved potentials, for example in relation to the behavior of the suckling, or to changes in parameters that require certain measurement frequencies or timings. Measurements from sensors **101, 102, 103, 104** may be utilized to set different measurement parameters such as characteristics of the injected current; or the number and positions of electrodes **100.**

According to some embodiments of the invention, measurements may be stored partly or fully, and be partly or fully used for calculations and later statistical analysis. Either raw data or processed data may be used for the algorithms.

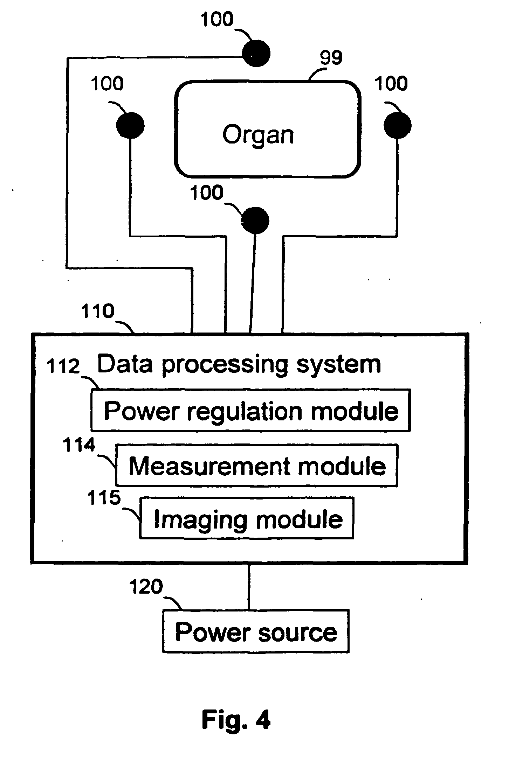

Fig. 4 is an illustration of a device for measuring characteristics of fluid excreted from an organ **99** of the human body, according to some embodiments of the invention. The device comprises at least one pair of electrodes **100,** a power source **120,** and a data processing system **110** comprising a power regulation module **112,** a measurement module **114,** and optionally an imaging module **115.** Electrodes **100** are connected to power source **120** via data processing system **110.** Power regulation module **112** regulates the current supplied to electrodes **100.** Data processing system **110** may comprise an external interface arranged to enable communication with the data processing system. The device may be constructed such as to be portable and simple to use.

Electrodes **100** may be arranged in pairs, and current may be directed to different pairs in relation to their position and in different temporal patterns. Electrodes **100** may be arranged in an array and be in contact with the human body in vicinity of fluid excreting organ **99.** The array may be characterized by a predefined spatial arrangement of electrodes **100** that is related to the form of fluid excreting organ **99.** Electrodes **100** may be arranged in different transecting planes in relation to organ **99,** around organ **99's** circumference etc., in ways that enhance the transmission, reception and analysis of the currents and in relation to the structure of organ **99.** Arrays may be chosen such that electrodes **100** are placed at plains representing cross section of organ **99.** The cross sections may be selected such as to enhance measuring characteristics of the excreted fluid.

Power regulation module **112** may be arranged to supply electrodes **100** with an electric current characterized by predefined parameters such as intensity patterns, frequency patterns, temporal patterns and spatial patterns, in relation to the predefined spatial arrangement of electrodes **100.** Power regulation module **112** may comprise a current generator and a multiplexer. According to some embodiments of the invention, power regulation module **112** may be arranged to supply electrodes **100** with an electric current characterized by parameters determined according to a predefined scheme, that may change periodically and show dependency on actual measurements. Parameters that may change include frequency, phase, amplitude and switching configuration. Electric currents characterized by different parameters may be injected into any number of electrodes **100** simultaneously. For example, different electrodes **100** may receive currents characterized by different frequencies, phases, or amplitudes. Any number of electrodes **100** may be used to inject the current, and their number may change during measurements. Measurements of evolved potentials may include data related to several currents and analysis may be carried out to differentiate the different donation, or components and thus accelerate and refine building the impedance images. Injected current may comprise a superposition of currents characterized by a range of frequencies, and analysis of evolved potentials may comprise reaction to the whole range of frequencies, a part of it, or single frequencies.

According to some embodiments of the invention, any electrode **100** may be utilized to either inject current, measure potentials, or both, according to a spatio temporal pattern.

According to some embodiments of the invention, data processing system **110** may further comprise a calibration module arranged to calibrate parameters relating to the device and to measurement module **114.**

Measurement module **114** may be arranged to measure characteristics of the excreted fluid from evolved potentials on the human body due to the electric current, in relation to the predefined spatial arrangement of electrodes **100** in the array. Measurement module **114** may comprise an analog amplifier arranged to amplify the reaction of organ **99** to the electric current, an analog to digital converter and a controller. Measurement module **114** may be arranged to measure characteristics of the excreted fluid from evolved potentials on the human body at predefined intervals, or in relation to former measurements or sensorial data. Differences between measurements may be used to calculate the amount of excreted fluid.

According to some embodiments of the invention, the device may utilize any number of electrodes **100** (e.g. one, two, three or more), depending on the size and form of organ **99** and the required accuracy of measurements. Pairs of electrodes **100** may be arranged such that some electrodes **100** deliver the current and other pairs of electrodes **100** measure the potential difference (from which impedance is calculated), or pairs of electrodes **100** may be utilized in an alternating manner (e.g. following a spatio-temporal pattern) to deliver current and to measure potential differences.

According to some embodiments of the invention, organ **99** may comprise a part that is constant in volume such as body tissue, and a part which changes its volume in organ **99,** such as excreted fluid. The evolved potentials may be related to the relative proportions of these two parts, and from measuring the evolved potentials, the volume and other characteristics (such as composition) of the two parts may be calculated. Special effort in the spatial arrangement and measurement algorithms is given to estimating the volume of the excreted fluid.

According to some embodiments of the invention, organ **99** may comprise a fluid excreting organ. According to some embodiments of the invention, organ **99** may comprise a breast, comprising biological tissues such as connecting tissues, glandular tissues, adipose tissues, as well as breast milk. The device may measure the volume and composition of breast milk before, during or after breast feeding. Electrodes **100** may be externally attached to the breastfeeding mother, preferably on her breast. Electrodes **100** may be attached at infant proximal (e.g. in the proximity of the nipple), medial or distal plains, or at a combination thereof.

According to some embodiments of the invention, the device may further comprise an apparatus arranged to support and place electrodes **100** in the array in contact of the human body in vicinity of organ **99,** or assist doing so.

According to some embodiments of the invention, the device may comprise any supporting element such as articles of clothing, garments or apparatuses to accommodate at least some of electrodes **100**, power source **120,** data processing system **110,** in a user friendly manner. Electrodes **100** may be located at different locations on the supporting element in relation to organ **99** to optimize signal transduction. Electrodes **100** may be connected between themselves and/or with a central electronic module. Connection could be through contact such as a wire, or contactless, such as radio frequency.

According to some embodiments of the invention, processing unit **80** may further comprise a calibration module, arranged to allow calibration of the device prior or during measurements. Calibration may be carried out in relation to measurements of known volumes of fluid (e.g. by measuring the volume of pumped milk). Calibration may be carried out once or repeatedly, and refer to different data relating to the lactation process, the mother and the baby as well as to statistical data relating to them. Calibration may be carried out using an empirical calibration instrument.

According to some embodiments of the invention, processing unit **80** may be arranged to form an impedance image of organ **99** by measuring evolved potentials at different cross-sections and different compartments.

According to some embodiments of the invention, processing unit **80** may be arranged to generate suggestions relating to possible improvement of the position of electrodes **100,** and to present the suggestions via user interface **90.** Processing unit **80** may be arranged to detect and inform of inappropriate or ineffective placing of electrodes **100** such as characterized by a low conductivity between the body and the electrode, or erroneous location in relation to **organ 99.**

According to some embodiments of the invention, user interface **90** may be arranged to present the measured evolved potentials, impedance, or the calculated characteristics to the user in a graphical or acoustical manner. User interface **90** may be arranged to present an impedance image of organ **99,** the volume of the milk (current or excreted), the composition of the milk and its nutritional value as well as data relating to recommended milk consumption of the baby (in relation to age, weight and baby data), sensorial data, time etc. In case other fluids are-measured, information may be presented relating to their concentration, volume, and composition. Further data may comprise estimated characteristics of the lactation process such as expected duration of milk volume, as well as current and expected caloric intake of the baby.

According to some embodiments of the invention, storage **86** may be utilized to save statistical data relating to former measurements (or measurements relating to other users). These data may be used to improve calculations, presentation and the measurement process itself (e.g. by comparing the efficiency of different arrays of electrodes **100**). Different data may be inputted and outputted via external interface **93.**

According to some embodiments of the invention, electrodes **100** may comprise different materials and designs, and may be attached to organ **99** in different ways. Electrodes may be designed in a manner making their placement intuitive and adaptable to organs **99** of different dimensions. The form of electrode array may be arranged in relation to the required results, e.g. in a two dimensional arc, in a three dimensional array etc. At least one of electrodes **100** is placed in the vicinity of organ **99.** Sensors' unit **70** may comprise additional sensors such as temperature sensors, optical and acoustical sensors to improve and correlate measurements.

According to some embodiments of the invention, data processing system **110** may further comprise imaging module **115** arranged to reconstruct at least one impedance image from the measured evolved potentials on the human body due to the electric current. Imaging module **115** may be further arranged to reconstruct at least one corrected impedance image from the measured evolved potentials as well as from the measurements from sensors **101, 102, 103, 104.** Imaging module **115** may further utilize calibration data relating to the measured evolved potentials or relating to the measurements from sensors **101, 102, 103, 104.** Imaging module **115** may further utilize statistical data relating to the measured evolved potentials or relating to the measurements from sensors **101, 102, 103, 104.** Imaging module **115** may further utilize statistical data relating to similar measurements with other devices, literature data etc.

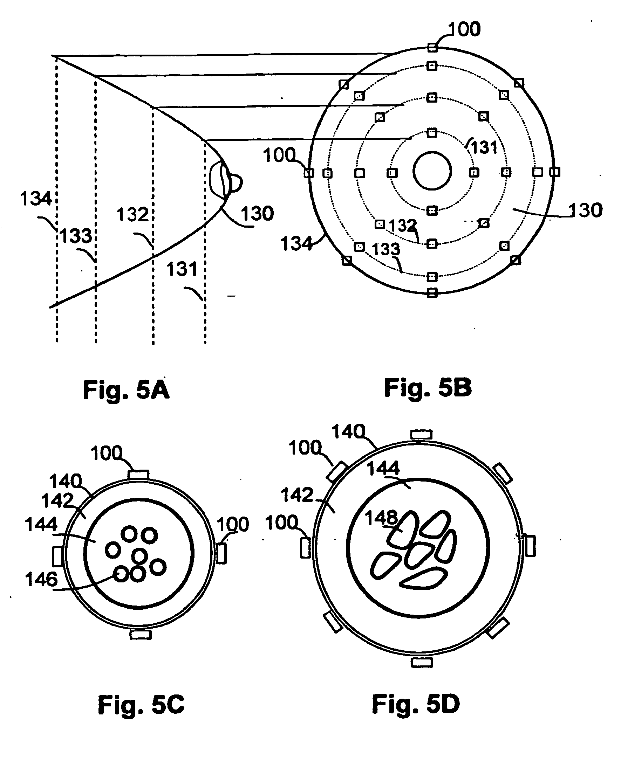

Figures 5A and 5B are illustrations of possible placement locations for electrodes **100** on a lactating breast, according to some embodiments of the invention. Fig. 5A is a side view of a breast **130** with transecting plains, Fig. 5B is a top view of breast **130** with transecting plains and electrodes **100** placed along the circumference of the intersections of the plains with breast **130.** Electrodes **100** may be attached on a breast **130** at infant proximal **131** (e.g. in the proximity of the nipple), different medial **132, 133** or distal plains **134,** or at a combination thereof. Electrodes **100** may be placed on different positions on the circumference of the intersections of plains **131, 132, 133, 134** with breast **130.** Electrodes **100** may be attached on breast **130,** e.g. using stickers, at some or all of the illustrated positions. The means for attaching electrodes **100** on breast **130** may comprise disposable or reusable stickers, or by applying a pressure using a garment.

Figures 5C and 5D are illustrations of schematic cross-sections of the lactating breast, showing possible placement of electrodes **100,** according to some embodiments of the invention. Figures 5C and 5D illustrate cross-sections at different plains of the breast, and present different proportions of skin **140,** fat **142,** connective tissue **144** with blood vessels and intraglandular fat, ducts **146** and alveoli and lobules **148** (see Fig.1 for comparison). Each element, or compartment, of the breast is characterized by different impedances, reflected in the impedance image. Monitoring the impedances of each element allows identifying changes relating to production and movement of milk among the elements, or compartments. The different proportions of these elements of the breast relate to different impedances. They further provide a possibility of calibrating the device for measuring characteristics of fluid excreted from the breast, by comparing measured values with stored data relating to similar cross-sections.

There are several parameters that affect the impedance of the lactating breast. Impedance of milk (contained in lobules **148** and ducts **146,** also 64 in Fig. 1) is small compared to impedance of adipose (fatty) tissue **142, 54.** Lobules and ducts impedance changes substantially during (and after) breastfeeding, due to the changing volume of contained milk. Milk is produced and contained in lobules **148** (glandular tissue **66**), while ducts **146** only store and convey the milk. Lobules **148** are located in the rear part of the breast, while the largest ducts **146** are located near the periphery or outer area of the breast. It may be important to separate out the part of the impedance due to every section. In addition to the techniques used in bulk measurements of impendance, impedance imaging may also be useful for separating out the different contributions to the impedance. The bulk measurements of potentials and impedance may be used to produce a map of the internal impedance of the organ, using various mathematical techniques of image reconstruction.

According to some embodiments of the invention, reconstructing an impedance image relies on a formulation of an initial impedance image. Formulating an initial impedance image of the breast comprises ascribing typical impedances to different compartments of the breast according to at least one image of the breast, and/or according to a model image of the breast (as in Figures 5C and 5D).

According to some embodiments of the invention, calculations may be carried out using the bulk measurements without generating a full impedance image.

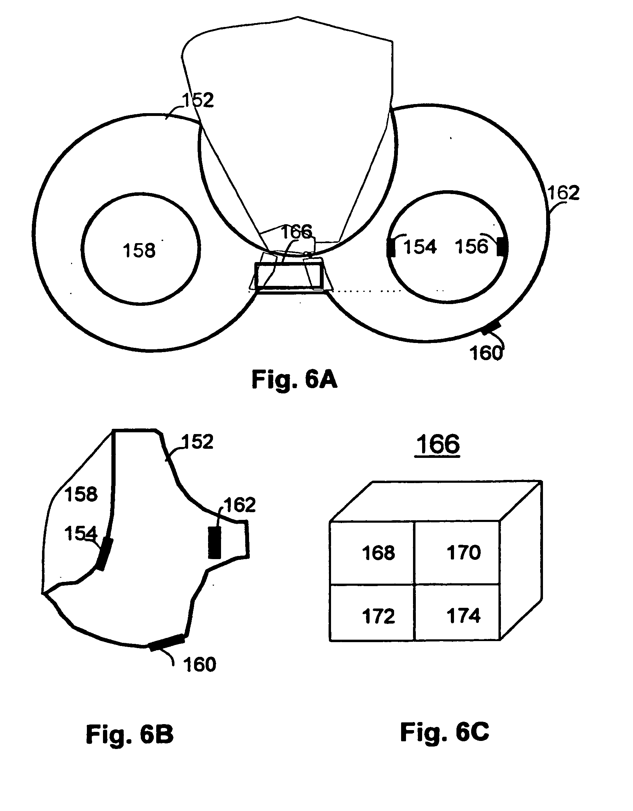

Figures 6A and 6B are illustrations of an apparatus arranged to support and place electrodes on a breast, according to some embodiments of the invention. Fig. 6A is a front view, Fig. 6B is a side view. The apparatus may comprise a standard or tailored brassiere **152** with a sufficient opening **158** for feeding the baby without its removal. The apparatus may comprise different models of nursing brassieres available on the market, as well as on other garments or apparatuses which do not serve the purpose of breast support. Brassiere **152** comprises electrodes which are located on one or more places on the brassiere such as: electrodes **154, 156** near the breast feeding opening, electrode **160** at the lower section of the brassiere's breast cup, electrode **162** on the side of the brassiere's breast cup, at the breast feeding distal section of the cup, or any combination of the above or at any other location on the breast feeding mother. The electrodes **154, 156, 160, 162** are connected between themselves and/or with a central electronic module **166.** Connection could be through contact such as a wire, or contactless, such as radio-frequency.

Fig. 6C is an illustration of central electronic module **166,** according to some embodiments of the invention. Electronic module **166** may comprise a power supply unit **168** of the device, an electronic signal generator **170** which is connected to the electrodes, a signal/ data acquisition and processing module **172,** and an input/output module **174.**

According to some embodiments of the invention, the device illustrated in Fig. 2 may further comprise brassiere **152** with a sufficient opening **158** for feeding the baby without removing brassiere **152.** At least some of electrodes **100,** power source **120,** data processing system **110** may be supported by brassiere **152.** Electrodes **100** may be located at one or more places on brassiere **152** such as near the breast feeding opening **156;** at the lower section of the brassiere's breast cup **160;** at the breast feeding distal section of the cup **162;** or any combination of the above or at any other location on the breast feeding mother. Electrodes **100** may be connected between themselves and/or with a central electronic module **176.** Connection could be through contact such as a wire **174,** or contactless, such as radio-frequency. Brassiere **152** may comprise any models of nursing brassieres available on the market.

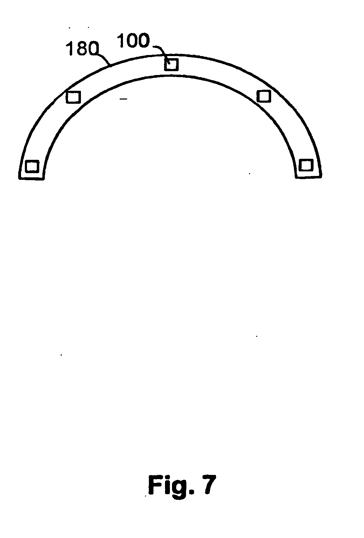

Fig. 7 is a cross section illustration of an appliance **180** for attaching electrodes to a breast, according to some embodiments of the invention. Electrodes **100** may be attached to appliance **180** such that they contact the breast while baby feeding. Appliance 180 may be applied on its own (e.g., an electrode patch), or in connection to a brassiere such as a nursing brassiere or a different garment.

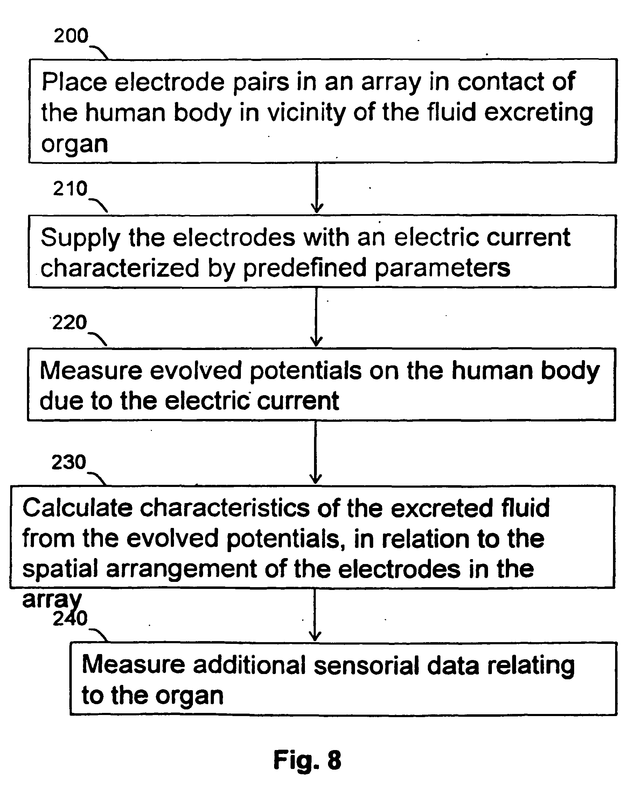

Fig. 8 is a flowchart illustrating a method of measuring characteristics of fluid excreted from an organ of the human body, according to some embodiments of the invention. The method comprises the stages: Placing one or more electrodes in an array in contact of the human body, where at least one electrode is in vicinity of the fluid excreting organ (stage **200**); supplying the electrodes with an electric current characterized by predefined parameters (stage **210**); measuring evolved potentials on the human body due to the electric current (stage **220**); and calculating characteristics of the excreted fluid from the measured evolved potentials in relation to the spatial arrangement of the electrodes in the array and possible prior data (stage **230**).

According to some embodiments of the invention, placing electrodes in an array (stage **200**) may follow predefined spatial arrangement, related to the form and size of the fluid excreting organ. At least one of the electrodes is placed in the vicinity of the organ. Placing electrodes (stage **200**) may be carried out in different transecting planes in relation to the organ, around the organ's circumference etc., in ways that enhance supplying current (stage **210**), measuring evolved potentials (stage **220**) and calculating characteristics of the excreted fluid from the measured evolved potentials (stage **230**).

According to some embodiments of the invention, placing electrodes in an array (stage **200**) may be carried out in relation to a supporting element such as a piece of clothing or an apparatus. For example, electrodes may be placed within a breastfeeding brassiere and calculating characteristics of the excreted fluid from the measured evolved potentials in relation to the spatial arrangement of the electrodes in the array (stage **230**) may relate to the quantity and quality of breast milk in the breast of a breast feeding woman.

According to some embodiments of the invention, placing electrodes in an array (stage **200**) may be carried out by attaching (e.g: gluing) the electrodes to the body. Placing electrodes (stage **200**) and later removing them may be carried out every measurement, or the electrodes may be worn for longer periods of time. Placing electrodes (stage **200**) may be carried out by integrating the electrodes into an apparatus, a brassiere or any other piece of clothing or garment, such that wearing these places the electrodes in the appropriate location.

According to some embodiments of the invention, placing electrodes in an array (stage **200**) may comprise choosing the array such that the electrodes are placed at plains representing predefined cross sections of the organ. The predefined cross section may be selected such as to enhance calculating characteristics of the excreted fluid from the measured evolved potentials (stage **230**).

According to some embodiments of the invention, supplying the electrodes with an electric current (stage **210**) may be carried out in different spatial and temporal patterns. Supplying current (stage **210**) and measuring evolved potentials (stage **220**) may be carried out with the same or different pairs of electrodes, and different patterns may govern exchanging electrode roles between supplying (stage **210**) and measuring (stage **220**). Supplying the electrodes with an electric current (stage **210**) may be carried out according to predefined parameters such as intensity patterns, frequency patterns, temporal patterns and spatial patterns, in relation to the predefined spatial arrangement of electrodes.

According to some embodiments of the invention, supplying the electrodes with an electric current characterized by predefined parameters (stage **210**) may comprise supplying the electrodes with an electric current characterized by parameters determined according to a predefined scheme, that may change periodically and show dependency on actual measurements. Parameters that may change include frequency, phase, amplitude and switching configuration. Supplying the electrodes with an electric current (stage **210**) may be carried out by injecting electric currents characterized by different parameters into any number of electrodes simultaneously. For example, different electrodes may receive currents characterized by different frequencies, phases, or amplitudes. Any number of electrodes may be used to inject the current, and their number may change during measurements. Measuring evolved potentials (stage **220**) may include data related to several currents and analysis (including stage **230**) may be carried out to differentiate the different donation, or components and thus accelerate and refine building the impedance images. Injected current may comprise a superposition of currents characterized by a range of frequencies, and analysis of evolved potentials may comprise reaction to the whole range of frequencies, a part of it, or single frequencies.

According to some embodiments of the invention, the organ may comprise a part that is constant in volume such as body tissue, and a part which changes its volume in organ, such as excreted fluid. Calculating characteristics of the excreted fluid from the measured evolved potentials in relation to the spatial arrangement of the electrodes in the array (stage **230**) may be aimed at estimating the relative proportions of these two parts, their volume and other characteristics (such as composition).

It may be striven to place electrodes (stage **200**) and to measure evolved potentials (stage **220**) such that estimating the volume of the excreted fluid is carried out in a high accuracy.

According to some embodiments of the invention, measuring evolved potentials of the human body to the electric current (stage **220**) may -be carried out utilizing different methods such as electrical impedance tomography by evaluating cross-sections of the organ or part of body measured, or compartment analysis focusing on different compartments of the measured organ. Measuring evolved potentials on the human body due to the electric current (stage **220**) may be carried out utilizing at least some of the electrodes to which electric current was supplied (stage **210**). Supplying current (stage **210** and measuring evolved potentials (stage **220**) may be carried out utilizing at least some common electrodes, and supplying current (stage **210** and measuring evolved potentials (stage **220**) may be interchanged in space and time at different, predefined patterns.

According to some embodiments of the invention, measuring evolved potentials on the human body due to the electric current (stage **220**) may be carried out at predefined intervals, or in relation to former measurements or sensorial data. Differences between measurement may be utilized to calculate the amount of the excreted fluid.

According to some embodiments of the invention, the method may further comprise measuring additional sensorial data relating to the organ of the human body (stage **240**). The sensorial data may comprise at least one of: Temperature measurements (e.g. surface temperature of the organ, temperature of the excreted fluid), mechanical measurement (e.g. abrupt movements of the organ, contractions of the organ), optical measurements (e.g.. changes in color, movements), acoustic measurement (e.g. gulps or suction sounds). Measuring the evolved potentials (stage **220**) may be carried out in relation to the additional sensorial data, e.g. initiated upon certain sensorial data, the frequency of measurements may depend on sensorial data, each single measurement may depend on sensorial data. For example, detection of baby gulps may require a higher measurement frequency. Calculating characteristics of the excreted fluid (stage **230**) may utilize the additional sensorial data.

According to some embodiments of the invention, measuring evolved potentials on the human body due to the electric current (stage **220**) may further comprise using AC bridges, lock-in amplifiers, LCR meters (LCR stands for Inductance (denoted by L), Capacitance and Resistance), impedance analyzer and similar appliances for detecting and analyzing the effects of the injected electric currents.

According to some embodiments of the invention, calculating characteristics of the excreted fluid from the measured evolved potentials in relation to the spatial arrangement of the electrodes in the array (stage **230**) may be carried out by relating the evolved potentials or differences in the measured evolved potentials to changes in the volume of the fluid in the organ or body part, based on calibration and statistical analysis or results. Measuring evolved potentials at different configurations of the electrodes and with different current characteristics allows constructing an impedance image of the measured organ or body part. Calculation then relates the impedance image to the volume and composition of the fluid in the body at different times and their changing during different time intervals. Calculation may incorporate measurements with other sensors, such as acoustic, optical or other sensors. Measurement, imaging and calculation may be carried out partially or fully in a parallel manner allowing feed-backing calculation results to measurements taking place. Calibration may be carried out using an empirical calibration instrument.

According to some embodiments of the invention, calculating characteristics of the excreted fluid from the measured evolved potentials (stage **230**) may comprise reconstructing at least one impedance image from the measured evolved potentials on the human body due to the electric current (stage **220**). Calculating characteristics of the excreted fluid from the measured evolved potentials (stage **230**) may further comprise reconstructing at least one corrected impedance image from at least one of the following data sources: the measured evolved potentials (stage **220**); the measured additional sensorial data (stage **240**); calibration data relating to either the measured evolved potentials (stage **220**) or the measured additional sensorial data (stage **240**); and statistical data relating to either the measured evolved potentials (stage **220**), the measured additional sensorial data (stage **240**), or external statistical data.

According to some embodiments of the invention, calculations may be utilized to generate suggestions relating to possible improvement of placing the electrodes. Suggestions regarding placing electrodes (stage **200**) may be presented during measurements, wherein their adoption may improve the accuracy of the measurements.

According to some embodiments of the invention, the method may further comprise detecting and informing of inappropriate or ineffective placing of electrodes (stage **200**) such as characterized by a low conductivity between the body and the electrode, or erroneous location in relation to the fluid excreting organ.

According to some embodiments of the invention, calculated characteristics of the excreted fluid (stage **230**) may comprise the volume of the excreted fluid and/or the composition of the excreted fluid.

According to some embodiments of the invention, the method may further comprise calibrating the measuring equipment. Calibration may be carried out once, e.g. by measuring a breast full of milk as a reference, or calibration may be carried out repeatedly after a predefined number of measurement cycles. Calibration may alternatively be carried out by pumping out a predefined or measureable amount of milk e.g. using a milk pump, and calibrating the measuring equipment accordingly. Calibration may be supported by different statistical data (e.g., data taken from one or more other nursing mothers and/or babies). Calibrating the measuring equipment may comprise inputting data such as relating to lactation times, durations and frequency and data relating to the mother and to the baby (such as age; weight, breast size, number or children and so on).

According to some embodiments of the invention, the method may further comprise applying electrical voltage to the electrodes.

According to some embodiments of the invention, the method may further comprise presenting the measured evolved potentials (stage **220**), impedances or the calculated characteristics (stage **230**). These may be presented graphically, during or after lactation. The presented values may comprise the evolved potentials, the impedance, the volume of the milk, the composition of the milk and its nutritional value. In cases other fluids are measured, information may be presented relating to their concentration, volume, and composition.

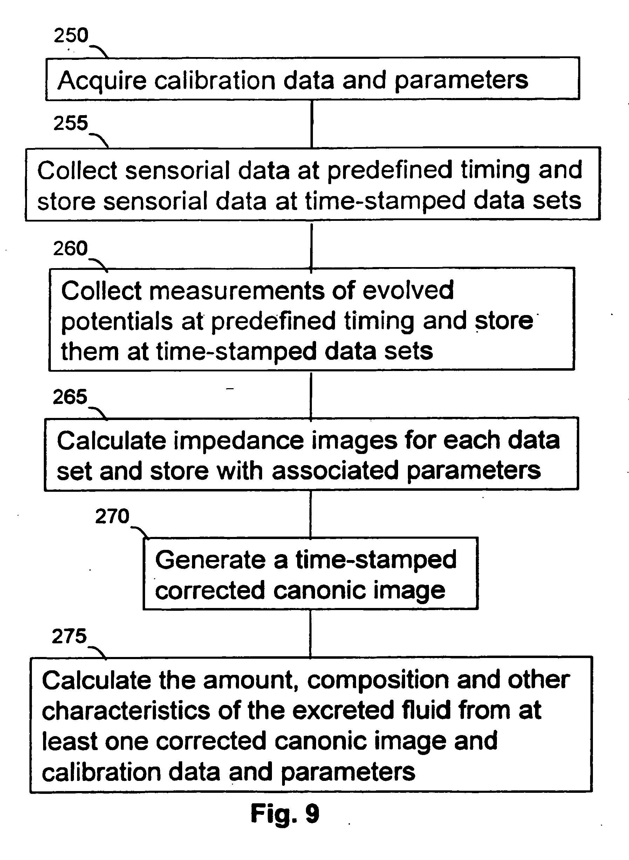

Fig. 9 is a flowchart illustrating a method of measuring characteristics of fluid excreted from an organ of the human body, according to some embodiments of the invention. The method comprises the stages: acquiring calibration data and parameters (stage **250**); collecting sensorial data at predefined timing and store sensorial data at time-stamped data sets (stage **255**); collecting measurements of evolved potentials on the human body due to the electric current at predefined timing and store the measurements of evolved potentials at time-stamped data sets (stage **260**); calculating impedance images for each data set and store with associated parameters (stage **265**); generating a time-stamped corrected canonic image (stage **270**); and calculating the amount, composition and other characteristics of the excreted fluid from at least one corrected canonic image and calibration data and parameters (stage **275**).

According to some embodiments of the invention, collecting sensorial data (stage 255) and collecting measurements of evolved potentials (stage **260**) may be carried out at predefined intervals, or in relation to former measurements or sensorial data, such that these allow the detection of volume changes in the organ. The predefined intervals and predefined timings may be such that some of the sensorial data and the measurements of evolved potentials represent static measurements (e.g. at short intervals) and others represent dynamic measurements (e.g. at longer intervals). Intervals and timing in general may be adjusted according to the measurement values and their analysis, adapting to the accuracy of measurements and the pace of the measured processes. Some of the measurements and data may be averaged and taken as base values. The timings of collecting sensorial data (stage **255**) and of collecting measurements of evolved potentials (stage **260**) may be interrelated or interdependent, partly or fully (e.g. sensorial data initiating measurements of evolved potentials). Collecting sensorial data (stage **255**) and of collecting measurements of evolved potentials (stage **260**) may be carried out before, during and after fluid excretion (e.g. breastfeeding).

According to some embodiments of the invention, calculating impedance images (stage **265**) and generating a time-stamped corrected canonic image (stage **270**) may utilize the measurements of evolved potentials and sensorial data, as well as the acquired calibration data and parameters (stage **250**) and predefined algorithms, constants, and functions. The images may be related to a certain patient by taking earlier measurements, be adjusted during measurements, or be based on statistical values. The terms image and canonic image do not necessarily relate to visual images, but represent the data generated by different measurements, at different cross-sections and plains. These data may be interpreted as a visual image in relation to the measured organ (e.g. a breast) or parts of it, such that different images convey different information. Calculation may be carried out using the bulk measurements without generating a full impedance image.

According to some embodiments of the invention, each calculated impedance image (stage **265**) and each generated time-stamped corrected canonic image (stage **270**) may be related or may comprise sensorial data with a similar time stamp. The time-stamped corrected canonic images may each represent the full stand of information relating to the organ at the stamped time or processed data relating to a temporal interval around the time stamp.

According to some embodiments of the invention, the impedance images and the I canonic images may be calculated (stage **265**) and generated (stage **270**) by solving a set of equations directly, reversely or iteratively, utilizing a combination of methods. Generating and correcting a canonic image (stage **270**) may be derived from the calculated impedance image (stage **265**) utilizing additional data (e.g. sensorial data, calibration data, statistical data, etc.), compression algorithms, algorithms for enhancing accuracy and learning.

According to some embodiments of the invention, the impedance image and the canonic images may be calculated and generated by solving the forward problem, i.e., find the potential distribution in the organ due to known current sources and impedance distribution, together with boundary conditions. Alternatively or additionally, said images may be calculated and generated by solving the inverse problem, i.e., find the impedance distribution with known potential distribution on the organ's surface and known current sources. Alternatively or additionally, said images may be calculated and generated by iterating solutions of the forward problem and inverse problem. Solving the inverse problem and/or forward problem may utilize a combination of methods.

According to some embodiments of the invention, collecting measurements of evolved potentials (stage **260**) may be carried out according to different current parameters and different times or simultaneously. For example a current comprising multiple superposed frequencies may be used and the voltage responses to the different frequencies may be separately analyzed and then combined.

According to some embodiments of the invention, calculating parameters of the fluid excreted (stage **275**), such as amount, volume, composition, may be carried out utilizing any number of time-stamped measurements, images and corrected canonic images. Algorithms may relate to any data on their own, as well as to any comparison of data sets.

The corrected canonic image may be calculated from the impedance image (stage **265**) or directly from raw data (stage **260**). The stages of the computer implemented method may be carried out in different order, and some stages may be skipped according to requirements.

According to some embodiments of the invention, measurements may be stored (stages **255**, **260**) partly or fully, and be partly or fully used for calculations and later statistical analysis. Either raw data or processed data may be used for the algorithms.

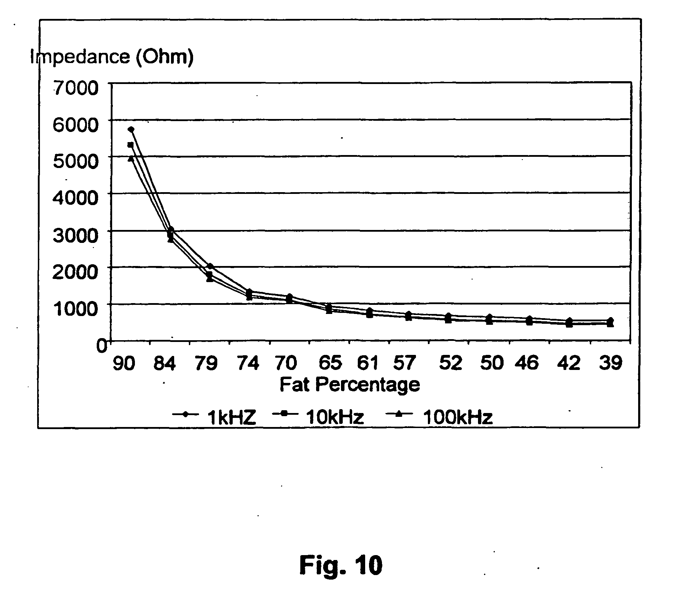

Fig. 10 is a graph summarizing bio-impedance measurements in fat emulsion models, according to some embodiments of the invention. The breast was modeled as an emulsion of animal fat and water places in a container. Different proportions of fat and water were achieved by changing the volume of water in the emulsion. The amount of fat in the emulsion was kept constant, while fat proportion in the emulsion was between 39% and 90% (x axis), simulating a lactating breast Bio-impedance was measured using copper electrodes attached to the sides of the container. Fig. 10 presents results of bio-impedance measurements with two electrodes, using three different frequency of an AC current applied to the electrodes: 1kHz, 10kHz and 100kHz represented by diamonds, squares and triangles, respectively. As presented in Fig. 10, bio-impedance measurements are sensitive to the percent of fat in the emulsion. Also, bio-impedance measurements of the fat emulsion in fixed fat percentages showed repeatability. According to initial measurements, the following characterizes the amount of milk in the breast and the related impedance and measurement accuracy. During breast feeding, the amount of milk in the breast decreases, and the impedance increases accordingly. As milk is excreted, new milk is generated, causing an decreasing component in the impedance, superposed over the general increase. The baby takes in the milk in a pulsating manner, creating recurring periods of decreasing amount of milk, causing the graphs of milk amount and impedance to be characterized by stepwise curves. In cross-sections, the impedance is likewise reciprocal to the amount of milk in the cross-section, allowing a spatial and temporal resolution to the impedance measurements. Additionally, initial breastfeeding is characterized by a gradual increase in milk production. The amount of milk generated in the breast increases with the amount of excreted milk, so that temporally the amount of milk in the breast may rise, with decreasing impedance measurements.

According to some embodiments of the invention, the device and method may be used to controlling and documenting the feeding of premature babies. Specifically, the device and method may allow controlling the amount and composition of milk received by premature babies while breastfed, and thus allow breastfeeding even to premature babies (which require a closer control of nutrition than other babies).

Electronic measurement has the following advantages over mechanical measurements: They do not separate the mother from the baby and thus do not disturb the natural process of breastfeeding, there is no need of disinfection, as the device does not contact the milk, electrical devices are more reliable than mechanical ones and are safe to operate and easy to operate and manipulate.

Bio-Impedance measurement has the following advantages over methods using inductance (especially inductance coils): They do not require applying strong magnetic fields, they are less sensitive to electromagnetic and environmental disturbances, and thus have a better signal to noise ratio, and are less sensitive to misplacing the electrodes, while measurement with inductance coils is very sensitive to movements of the coils. In addition, further advantages comprise safety through using AC currents with a low amplitude and avoiding strong magnetic fields or ionizing radiation - thus allowing measurements over long periods of time; the possibility of generating impedance images of different plains and compartments, allowing electrical impedance tomography; and utilizing cheaper hardware and allowing the device to be easily transportable.

According to some embodiments of the invention, the use of BIA technology allows safe measurement of the volume of the nursed breast-milk during a lactation session with no dividing "buffer" between the mother and her baby. The avoidance of having a divider between the mother and her baby has many advantages, such as the following. Cleansing - The device does not come in contact with the breast-milk, and thus spares the need for cleansing the device (or dividing "buffer") after every use. Baby's safety - The baby nurses his milk in the natural way directly from the nipple. Thus, there are no mechanical objects or barriers he might accidentally swallow. Convenience - The lack of any divider between the mother and the baby is a main factor that contributes to the comfort of using the device. Natural process - Having no divider, leaves the breastfeeding procedure natural with no interference to the babies (or breast's) suction ability, and keeps the basic body instincts natural. Also, having no barrier between the mother and her baby does not impair the psychological maternal bonding. Reliability - The device is highly reliable due to the lack of any moving mechanical parts. Regulatory **-** BIA is a known technology approved to use on people by the FDA (U.S. Food and Drug Administration) and other regulatory agencies.

In the above description, an embodiment is an example or implementation of the inventions. The various appearances of "one embodiment," "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

It is understood that the phraseology and terminology employed herein is not to be construed as limiting and are for descriptive purpose only.

The principles and uses of the teachings of the present invention may be better understood with reference to the accompanying description, figures and examples.

It is to be understood that the details set forth herein do not construe a limitation to an application of the invention.

Furthermore, it is to be understood that the invention can be carried out or practiced in various ways and that the invention can be implemented in embodiments other than the ones outlined in the description above.

It is to be understood that where the claims or specification refer to "a" or "an" element, such reference is not be construed that there is only one of that element.

It is to be understood that where the specification states that a component, feature, structure, or characteristic "may", "might", "can" or "could" be included, that particular component, feature, structure, or characteristic is not required to be included.

Where applicable, although state diagrams, flow diagrams or both may be used to describe embodiments, the invention is not limited to those diagrams or to the corresponding descriptions. For example, flow need not move through each illustrated box or state, or in exactly the same order as illustrated and described.

Methods of the present invention may be implemented by performing or completing manually, automatically, or a combination thereof, selected steps or tasks.

The term "method" may refer to manners, means, techniques and procedures for accomplishing a given task including, but not limited to, those manners, means, techniques and procedures either known to, or readily developed from known manners, means, techniques and procedures by practitioners of the art to which the invention belongs.

The descriptions, examples, methods and materials presented in the claims and the specification are not to be construed as limiting but rather as illustrative only.

Meanings of technical and scientific terms used herein are to be commonly understood as by one of ordinary skill in the art to which the invention belongs, unless otherwise defined.

The present invention can be implemented in the testing or practice with methods and materials equivalent or similar to those described herein.

While the invention has been described with respect to a limited number of embodiments, these should not be construed as limitations on the scope of the invention, but rather as exemplifications of some of the preferred embodiments. Those skilled in the art will envision other possible variations, modifications, and applications that are also within the scope of the invention. Accordingly, the scope of the invention should not be limited by what has thus far been described, but by the appended claims and their legal equivalents.

## Claims

1. A device for measuring characteristics of fluid excreted from an organ of the human body, the device comprising:
at least one pair of electrodes (100);
a power source; and
a data processing system(110) comprising:
a power regulation module(112); and
a measurement module(114),
wherein the at least one pair of electrodes (100) are connected to the power source(87) via the data processing system;
wherein the at least one pair of electrodes (100) are arranged in an array in contact of the human body, the array **characterized by** a predefined spatial arrangement of at least two dimensional geometrical space of the at least one pair of electrodes that is related to the three dimension geometrical form of the fluid excreting organ, and wherein at least one electrode is placed in the vicinity of the fluid excreting organ;
wherein the power regulation module is arranged to supply the at least one pair of electrodes (100) with an electric current **characterized by** predefined parameters;
and wherein the measurement module is arranged to determine the characteristics of the excreted fluid and the volume change of the excreting organ from evolved potentials on the human body due to the electric current, in relation to the predefined spatial arrangement of the at least one pair of electrodes in the array (100).

2. The device of claim 1, further comprising at least one of additional sensors:
acoustic sensor (103); optical sensor (101); and temperature sensor (104), wherein the data processing (110) system is arranged to enhance the measurement of the characteristics of the excreted fluid utilizing data from the additional sensors,

3. The device of claim 2, wherein the data processing (110) system further comprises an imaging module arranged to reconstruct at least one corrected impedance image from the evolved potentials on the human body due to the electric current, and at least one of: data from the at least one of additional sensors, calibration data relating thereto, statistical data relating thereto.

4. The device of claim 1, further comprising an apparatus (152) arranged to support and place the at least one pair of electrodes (100) in the array in contact of the human body in vicinity of the fluid excreting organ.

5. The device of claim 1, wherein the array is chosen such that the electrodes (100) are placed in at least one plane representing a cross section of the organ, wherein the cross section is selected such as to enhance measuring characteristics of the excreted fluid.

6. The device of claim 1, wherein the data processing system (110) further comprises a calibration module arranged to calibrate parameters relating to the device and to the measurement module.

7. The device of claim 1, wherein the data processing (110) system further comprises an imaging module (115) arranged to reconstruct at least one impedance image from the evolved potentials on the human body due to the electric current.

8. The device of claim 1, wherein the measurement module (114) is arranged to measure characteristics of the excreted fluid from the evolved potentials on the human body due to the electric current at least one of: predefined intervals, timing related to said measured characteristics.

9. The device of claim 1, wherein the measured characteristics of the excreted fluid comprise at least one of: volume of the excreted fluid, at least partial composition of the excreted fluid.

10. The device of claim 1, wherein the organ of the human body is a breast and the excreted fluid is breast milk, and wherein the device allows a breast feeding mother to control characteristic of fed breast milk.

11. A method of measuring characteristics of fluid excreted from an organ of the human body, the method comprising:
placing at least one pair of electrodes (100) in an array in contact of the human body;
supplying the at least one pair of electrodes (100) with an electric current **characterized by** predefined parameters;
measuring evolved potentials on the human body due to the electric current; and
calculating characteristics of the excreted fluid and the volume change of the excreting organ from the measured evolved potentials in relation to the spatial arrangement of the at least one pair of electrodes in the array,
wherein at least one electrode is placed in the vicinity of the fluid excreting organ.

12. The method of claim 11, wherein placing at least one pair of electrodes is carried out according to a predefined spatial arrangement related to the form and size of the fluid excreting organ, such that the predefined spatial arrangement enhances at least one of: said supplying current, said measuring evolved potentials, said calculating characteristics.

13. The method of claim 11, wherein the array is chosen such that the electrodes (100) are placed at at least one plane representing a predefined cross section of the organ, and wherein the predefined cross section is selected such as to enhance said calculating characteristics of the excreted fluid from the measured evolved potentials.

14. The method of claim 11, wherein the characteristics of the excreted fluid comprise at least one of volume of the excreted fluid, composition of the excreted fluid.

15. The method of claim 11, wherein said supplying the at least one pair of electrodes (100) with an electric current is carried out in predefined spatial and temporal patterns, and wherein said measuring of the evolved potentials on the human body due to the electric current is carried out at predefined intervals.

16. The method of claim 11, wherein the organ of the human body is a breast.

17. The method of claim 11, wherein said calculating characteristics of the excreted fluid comprises reconstructing at least one impedance image from the measured evolved potentials on the human body due to the electric current.

18. The method of claim 11, further comprising measuring additional sensorial data relating to the organ of the human body, wherein said sensorial data comprises at least one of: Temperature measurements, mechanical measurement, optical measurements, acoustic measurement.

19. The method of claim 18, wherein said measuring evolved potentials of the human body to the electric current and calculating characteristics of the excreted fluid is carried out in relation to said additional sensorial data.

20. The method of claim 18, wherein said calculating characteristics of the excreted fluid comprises reconstructing at least one corrected impedance image from the measured evolved potentials, and at least one of: the additional sensorial data, calibration data relating thereto, statistical data relating thereto.

21. The device of claim 1 wherein the measurement module (114) is further arranged to measure characteristics of the breast milk from the evolved potentials, on the human body due to the electric current at least one of predefined intervals, timing related to said measured characteristics, the user interface arranged to present at least one of: the measured characteristics, statistical data according to infant's age; and wherein the supporting apparatus is arranged to support and place the at least one pair of electrodes (100) in the array in contact of the human body in vicinity of the breast;

22. The device of claim 1 wherein the measured characteristics of the breast milk comprise at least one of: volume of the breast milk, composition of the breast milk; and wherein the array is chosen such that the electrodes (100) are placed at least one plane representing a cross section of the breast, wherein the cross section is selected such as to enhance measuring characteristics of the breast milk.

23. The device of claim 1 wherein the determination of the characteristics of the excreted fluid and the volume change of the excreting organ is in relation to the spatial arrangement of the electrodes, based on impedance characterizations of the organ and measured potentials..

## Patentansprüche

1. Eine Vorrichtung zum Messen von Merkmalen bzw. Charakteristika einer Flüssigkeit, die von einem Organ des menschlichen Körpers ausgeschieden wird, wobei die Vorrichtung umfasst:
zumindest ein Paar von Elektroden (100);
eine Leistungsquelle; und
ein Datenverarbeitungssystem (110) umfassend:
ein Leistungsregelmodul (112); und
ein Messmodul (114),
wobei das zumindest eine Paar von Elektroden (100) mit der Leistungsquelle (87) über das Datenverarbeitungssystem verbunden ist;
wobei das zumindest eine Paar von Elektroden (100) in einer Reihe in Kontakt mit dem menschlichen Körper angeordnet ist, wobei die Reihe charakterisiert ist durch eine vordefinierte räumliche Anordnung von einem zumindest zweidimensionalen geometrischen Raum des zumindest einen Paars von Elektroden, der auf die dreidimensionale geometrische Form des Flüssigkeit ausscheidenden Organs bezogen ist, und wobei zumindest eine Elektrode in der Nähe des Flüssigkeit ausscheidenden Organs platziert ist;
wobei das Leistungsregelmodul angeordnet ist, das zumindest eine Paar von Elektroden (100) mit einem elektrischen Strom, der charakterisiert ist durch vordefinierte Parameter, zu versorgen;
und wobei das Messmodul angeordnet ist, die Merkmale der ausgeschiedenen Flüssigkeit und die Volumenänderung des ausscheidenden Organs aus den in dem menschlichen Körper aufgrund des elektrischen Stroms entstandenen Potentialen zu ermitteln, in Bezug auf die vordefinierte räumliche Anordnung des zumindest einen Paars von Elektroden in der Reihe (100).

2. Die Vorrichtung nach Anspruch 1, weiter umfassend zumindest einen zusätzlichen der folgenden Sensoren:
einen akustischen Sensor (103); einen optischen Sensor (101); und einen Temperatursensor (104), wobei das Datenverarbeitungssystem (110) angeordnet ist, die Messung der Merkmale der ausgeschiedenen Flüssigkeit durch Nutzung der Daten der zusätzlichen Sensoren zu verbessern.

3. Die Vorrichtung nach Anspruch 2, wobei das Datenverarbeitungssystem (110) weiter ein Bildgebungsmodul umfasst, das angeordnet ist, zumindest ein korrigiertes Impedanzbild von den im menschlichen Körper aufgrund des elektrischen Stroms entstandenen Potentialen zu rekonstruieren, und zumindest einem der folgenden: Daten von dem zumindest einen zusätzlichen Sensor, darauf bezogene Kalibrierdaten, darauf bezogene statistische Daten.

4. Die Vorrichtung nach Anspruch 1, weiter umfassend einen Apparat (152), der angeordnet ist, das zumindest eine Paar von Elektroden (100) in der Reihe in Kontakt mit dem menschlichen Körper in der Nähe des Flüssigkeit ausscheidenden Organs abzustützen und zu platzieren.

5. Die Vorrichtung nach Anspruch 1, wobei die Reihe derart gewählt ist, dass die Elektroden (110) in zumindest einer Ebene platziert sind, die einen Querschnitt des Organs repräsentiert, wobei der Querschnitt so ausgewählt ist, dass das Messen der Merkmale der ausgeschiedenen Flüssigkeit verbessert wird.

6. Die Vorrichtung nach Anspruch 1, wobei das Datenverarbeitungssystem (110) weiter ein Kalibriermodul umfasst, das angeordnet ist, Parameter, welche sich auf die Vorrichtung und das Messmodul beziehen, zu kalibrieren.

7. Die Vorrichtung nach Anspruch 1, wobei das Datenverarbeitungssystem (110) weiter ein Bildgebungsmodul (115) umfasst, das angeordnet ist, zumindest ein Impedanzbild aus den im menschlichen Körper aufgrund des elektrischen Stroms entstandenen Potentialen zu rekonstruieren.

8. Die Vorrichtung nach Anspruch 1, wobei das Messmodul (144) angeordnet ist, Merkmale der ausgeschiedenen Flüssigkeit von den in dem menschlichen Körper aufgrund des elektrischen Stroms entstandenen Potentialen zumindest wie folgt zu messen: vordefinierte Intervalle, Takt bzw. Timing bezogen auf die gemessenen Merkmale.

9. Die Vorrichtung nach Anspruch 1, wobei die gemessenen Merkmale der ausgeschiedenen Flüssigkeit zumindest eines der folgenden umfassen:
Volumen der ausgeschiedenen Flüssigkeit, zumindest teilweise Zusammensetzung der ausgeschiedenen Flüssigkeit.

10. Die Vorrichtung nach Anspruch 1, wobei das Organ des menschlichen Körpers eine Brust und die ausgeschiedene Flüssigkeit Muttermilch ist, und wobei die Vorrichtung einer stillenden Mutter erlaubt, Merkmale der gefütterten Muttermilch zu steuern bzw. zu regeln.

11. Ein Verfahren zum Messen von Merkmalen einer Flüssigkeit, die von einem Organ des menschlichen Körpers ausgeschieden wird, wobei das Verfahren umfasst:
Platzieren von zumindest einem Paar von Elektroden (110) in einer Reihe in Kontakt mit dem menschlichen Körper;
Versorgen des zumindest einen Paars von Elektroden (110) mit einem elektrischem Strom, der charakterisiert ist durch vordefinierte Parameter;
Messen von in dem menschlichen Körper aufgrund des elektrischen Stroms entstandenen Potentialen; und Berechnen von Merkmalen der ausgeschiedenen Flüssigkeit und der Volumenänderung des ausscheidenden Organs aus den gemessenen entstandenen Potentialen in Bezug auf die räumliche Anordnung des zumindest einen Paars von Elektroden in der Reihe,
wobei zumindest eine Elektrode in der Nähe des Flüssigkeit ausscheidenden Organs platziert ist.

12. Das Verfahren nach Anspruch 11, wobei das Platzieren des zumindest einen Paars von Elektroden gemäß einer vordefinierten räumlichen Anordnung bezogen auf die Form und Größe des Flüssigkeit ausscheidenden Organs durchgeführt wird, so dass die vordefinierte räumlichen Anordnung zumindest eines der folgenden verbessert: das Versorgen mit Strom, das Messen der entstandenen Potentiale, das Berechnen von Merkmalen.

13. Das Verfahren nach Anspruch 11, wobei die Reihe derart gewählt ist, dass die Elektroden (100) in zumindest einer Ebene, die einen vordefinierten Querschnitt des Organs repräsentiert, platziert sind und wobei der vordefinierte Querschnitt derart gewählt ist, dass das Berechnen von Merkmalen der ausgeschiedenen Flüssigkeit aus den gemessenen entstandenen Potentialen verbessert wird.

14. Das Verfahren nach Anspruch 11, wobei die Merkmale der ausgeschiedenen Flüssigkeit zumindest eines der folgenden umfassen: Volumen der ausgeschiedenen Flüssigkeit, Zusammensetzung der ausgeschiedenen Flüssigkeit.

15. Das Verfahren nach Anspruch 11, wobei das Versorgen des zumindest einen Paars von Elektroden (110) mit einem elektrischen Strom in vordefinierten räumlichen und zeitlichen Mustern durchgeführt wird, und wobei das Messen der im menschlichen Körper aufgrund des elektrischen Stroms entstandenen Potentiale in vordefinierten Intervallen durchgeführt wird.

16. Das Verfahren nach Anspruch 11, wobei das Organ des menschlichen Körpers eine Brust ist.

17. Das Verfahren nach Anspruch 11, wobei das Berechnen von Merkmalen der ausgeschiedenen Flüssigkeit ein Rekonstruieren zumindest eines Impedanzbildes aus den in dem menschlichen Körper aufgrund des elektrischen Stroms entstandenen gemessenen Potentialen umfasst.

18. Das Verfahren nach Anspruch 11, weiter umfassend ein Messen zusätzlicher sensorischer Daten bezogen auf das Organ des menschlichen Körpers, wobei die sensorischen Daten zumindest eines der folgenden umfassen: Temperaturmessungen, eine mechanische Messung, optische Messungen, eine akustische Messung.

19. Das Verfahren nach Anspruch 18, wobei das Messen der in dem menschlichen Körper aufgrund des elektrischen Stroms entstandenen Potentiale und das Berechnen von Merkmalen der ausgeschiedenen Flüssigkeit in Relation zu den zusätzlichen sensorischen Daten durchgeführt wird.

20. Das Verfahren nach Anspruch 18, wobei das Berechnen von Merkmalen der ausgeschiedenen Flüssigkeit ein Rekonstruieren zumindest eines korrigierten Impedanzbildes aus den gemessenen entstandenen Potentiale umfasst, und zumindest eines der folgenden: die zusätzlichen sensorischen Daten, darauf bezogene Kalibrierdaten, darauf bezogene statistische Daten.

21. Die Vorrichtung nach Anspruch 1, wobei das Messmodul (114) weiter angeordnet ist, Merkmale der Muttermilch aus den in dem menschlichen Körper aufgrund des elektrischen Stroms entstandenen Potentialen zumindest gemäß einem der folgenden zu messen: vordefinierte Intervalle, Timing bezogen auf die gemessenen Merkmale, wobei die Benutzerschnittstelle angeordnet ist, zumindest eines der folgenden anzuzeigen:
gemessene Merkmale, statistische Daten gemäß dem Alter des Kindes; und wobei der Unterstützungsapparat angeordnet ist, das zumindest eine Paar von Elektroden (100) in der Reihe in Kontakt mit dem menschlichen Körper in der Nähe der Brust abzustützen und zu platzieren.

22. Die Vorrichtung nach Anspruch 1, wobei die gemessenen Merkmale der Muttermilch zumindest eines der folgenden umfassen: Volumen der Muttermilch, Zusammensetzung der Muttermilch; und wobei die Reihe derart gewählt ist, dass die Elektroden (100) in zumindest einer Ebene platziert sind, die einen Querschnitt der Brust repräsentiert, wobei der Querschnitt derart gewählt ist, dass das Messen von Merkmalen der Muttermilch verbessert wird.

23. Die Vorrichtung nach Anspruch 1, wobei die Bestimmung der Merkmale der ausgeschiedenen Flüssigkeit und die Volumenänderung des ausscheidenden Organs in Relation zu der räumlichen Anordnung der Elektroden erfolgt, basierend auf Impedanzcharakterisierungen des Organs und gemessener Potentiale.

## Revendications

1. Procédé de surveillance d'un service de réseau de communication, comprenant les étapes de :
- sélection d'au moins deux paramètres d'un réseau (P0, P1, P2), représentatifs d'un service réseau et variables dans le temps ;
- mesure et/ou calcul à au moins deux instants des valeurs des paramètres réseau;
- détermination à au moins deux instants de la valeur d'un indicateur de service (1, 4) en fonction desdites valeurs mesurées et/ou calculées des paramètres;
**caractérisé en ce qu'**il comprend en outre une étape de pondération des paramètres réseau en fonction du service à surveiller, l'indicateur de service étant déterminé en fonction desdites valeurs mesurées et/ou calculées des paramètres ainsi pondérés et une étape de détermination d'un indicateur de tendance de l'indicateur de service en fonction desdites valeurs d'indicateur de service ainsi déterminées, ledit indicateur de tendance étant propre à indiquer à un instant choisi l'évolution dans le temps dudit indicateur de service ainsi surveillé.

2. Le procédé de la revendication 1, **caractérisé en ce qu'**il comprend en outre une étape ultérieure de détermination en fonction de l'indicateur de tendance de l'indicateur de service d'un instant de franchissement (T1, T2) par le dit indicateur de service d'un seuil défini (2).

3. Le procédé de la revendication 1, **caractérisé en ce que** le service réseau est choisi dans le groupe constitué de la voix, du vidéophone, de la téléphonie, du multimédia, de la vidéo sur demande, du réseau privé virtuel, des données en temps réel, des données interactives et des flux de données.

4. Le procédé de la revendication 1, **caractérisé en ce qu'**il comprend en outre une étape de détermination d'un plan (1) comme indicateur en effectuant une régression linéaire sur les paramètres réseau mesurés et/ou calculés.

5. Le procédé de la revendication 1, **caractérisé en ce que** la détermination de l'indicateur de tendance de service comprend la comparaison de valeurs des paramètres à des seuils prédéterminés.

6. Le procédé de la revendication 5, **caractérisé en ce que** la détermination de l'indicateur de tendance de service comprend en outre
- la détermination de tendance d'un paramètre réseau ;
- la comparaison de la tendance du paramètre à un seuil prédéterminé.

7. Le procédé de la revendication 1, **caractérisé en ce que** la détermination de l'indicateur de tendance de l'indicateur de service est réalisée par un réseau neuronal (10).

8. Le procédé de la revendication 7, **caractérisé en ce qu'**il comprend en outre une étape d'apprentissage dans laquelle le réseau neuronal détermine des règles d'association entre un indicateur de tendance de service et des valeurs de paramètres de service.

9. Le procédé de la revendication 8, **caractérisé en ce que** l'étape d'apprentissage comprend l'insertion dans le réseau neuronal d'une base d'apprentissage présentant des valeurs d'indicateur de tendance de services associées à des valeurs de paramètres de service.

10. Le procédé de la revendication 9, **caractérisé en ce que** le réseau neuronal est un réseau multi-couches.

11. Le procédé de la revendication 1, **caractérisé en ce qu'**il comprend en outre une étape de calcul d'une espérance mathématique de perte financière en fonction de l'indicateur tendance de service réseau déterminée.

12. Le procédé de la revendication 1 **caractérisé en ce qu'**il comprend en outre une étape de détermination d'une capacité à un instant donné à fournir un service réseau.

13. Un système de gestion de réseau et/ou de services, **caractérisé en ce qu'**il met en oeuvre un procédé selon l'une des revendications précédentes.

14. Procédé selon la revendication 11, dans lequel les caractéristiques du fluide excrété comprennent au moins une parmi : volume du fluide excrété, composition du fluide excrété.

15. Procédé selon la revendication 11, dans lequel ladite alimentation de l'au moins une paire d'électrodes (100) avec un courant électrique est exécutée dans des schémas spatiaux et temporels prédéterminés, et dans lequel ladite mesure des potentiels développés sur le corps humain à cause du courant électrique est exécutée à des intervalles prédéterminés.

16. Procédé selon la revendication 11, dans lequel l'organe du corps humain est un sein.

17. Procédé selon la revendication 11, dans lequel ledit calcul de caractéristiques du fluide excrété comprend la reconstruction d'au moins une image d'impédance à partir des potentiels développés mesurés sur le corps humain à cause du courant électrique.

18. Procédé selon la revendication 11, comprenant en outre la mesure de données sensorielles additionnelles relatives à l'organe du corps humain, dans lequel lesdites données sensorielles comprennent au moins une parmi : des mesures de température, une mesure mécanique, des mesures optiques, une mesure acoustique.

19. Procédé selon la revendication 18, dans lequel ladite mesure de potentiels développés sur le corps humain à cause du courant électrique et ledit calcul de caractéristiques du fluide excrété sont exécutés en relation avec lesdites données sensorielles additionnelles.

20. Procédé selon la revendication 18, dans lequel ledit calcul de caractéristiques du fluide excrété comprend en outre la reconstruction d'au moins une image d'impédance corrigée à partir des potentiels développés mesurés et d'au moins une parmi : les données sensorielles additionnelles, des données d'étalonnage relatives à celles-ci, des données statistiques relatives à celles-ci.

21. Procédé selon la revendication 1, dans lequel le module de mesure (114) est agencé en outre pour mesurer des caractéristiques du lait maternel à partir des potentiels développés sur le corps humain à cause du courant électrique à au moins un parmi : des intervalles prédéterminés, une synchronisation liée auxdites caractéristiques mesurées, l'interface d'utilisateur étant agencée pour présenter au moins une parmi : les caractéristiques mesurées, des données statistiques en fonction de l'âge du nourrisson ; et dans lequel le dispositif de support est agencé pour supporter et positionner l'au moins une paire d'électrodes (100) dans la disposition en contact avec le corps humain à proximité du sein.

22. Procédé selon la revendication 1, dans lequel les caractéristiques mesurées du lait maternel comprennent au moins un parmi : volume du lait maternel, composition du lait maternel ; et dans lequel la disposition est choisie de manière que les électrodes (100) soient placées dans au moins un plan représentant une section transversale de l'organe, dans lequel la section transversale est choisie de manière à améliorer les caractéristiques de mesure du lait maternel.

23. Procédé selon la revendication 1, dans lequel la détermination des caractéristiques du fluide excrété et la variation de volume de l'organe d'excrétion est en relation avec l'agencement spatial des électrodes, sur la base de caractérisations d'impédance de l'organe et de potentiels mesurés.
